# EUROPEAN PATENT APPLICATION

(11) **EP 4 523 603 A1**
(43) Date of publication of application: **19.03.2025**
(21) Application number: 24200026.3
(22) Date of filing: 12.09.2024
(51) Int. Cl.: A61B 1/12, A61B 1/00, A61B 90/70

(54) **METHOD AND ARRANGEMENT FOR PRE-CLEANING AN ENDOSCOPE**

(30) Priority: 13.09.2023 US 202363538116 P
(71) Applicant: Olympus Winter & Ibe GmbH, 22045 Hamburg (DE)
(72) Inventor: Ottens, Benjamin, 22399 Hamburg (DE)
(74) Representative: Eisenführ Speiser

(57) **Abstract**

The invention relates to a method for pre-cleaning an endoscope. Further, the invention relates to an arrangement for pre-cleaning an endoscope. It is provided a method for pre-cleaning an endoscope, preferably immediately or shortly after use of the endoscope, the method comprising: providing an endoscope that comprises a channel system, the channel system comprising: a first channel section, a second channel section and a third channel section, wherein these channel sections are connect to each other at a channel connection, and a first channel opening, a second channel opening and a third channel opening; connecting a liquid source, in particular a vessel that holds liquid, a suction device, preferably a suction pump, and a sealing element each to a different one of the first, second, and third channel opening, wherein the liquid source has a holding chamber with a holding chamber volume that is changeable; activating the suction device for drawing liquid from the liquid source towards the suction device, wherein the holding chamber volume of the liquid source reduces, preferably by the volume of liquid that is drawn out of the liquid source, during drawing of liquid from the liquid source. This way, air can be drawn out of the channel section to which the sealing element is connected to by applying an underpressure using the suction device, whereby an underpressure can build up in this channel section and the other channel sections are filled with liquid, so that when in a further step the suction device is turned off and/or disconnected from the channel system due to the underpressure in the channel to which the sealing element is connected to liquid can be drawn into this channel section so that this channel section is also filled with the liquid.

## Description

### TECHNICAL FIELD

The invention relates to a method for pre-cleaning an endoscope. Further, the invention relates to an arrangement for pre-cleaning an endoscope.

### BACKGROUND

An endoscope is an inspection instrument that can be used to examine, and optionally manipulate, the inside of organisms. Endoscopes can be used in human medical diagnostics for examinations or minimally invasive surgical procedures on humans or animals. Endoscopes typically comprise an image sensor, an optical lens, and a light source.

After use of an endoscope, the endoscope is typically pre-cleaned, manually or automated cleaned, disinfected, and possibly sterilized before being used again on a patient. If an endoscope and accessories used in the patient procedure are not immediately cleaned after each patient procedure, residual organic debris will begin to dry and solidify, hindering effective removal and reprocessing efficacy. Therefore, it is necessary to pre-clean the endoscope and the accessories at the bedside immediately after each patient procedure.

Typically, the pre-cleaning that is conducted at the bedside immediately after each patient procedure inter alia comprises the following steps: First, a biopsy/suction channel is pre-cleaned by arranging a distal end of the endoscope in water, and drawing the fluid through the endoscope. Then, air is drawn through the endoscope. Second, an air/water channel is pre-cleaned by arranging the distal end of the endoscope in water and flush the air/water channel with the fluid. Then, the air/water channel is flushed with air. Third, if available, an auxiliary water channel is pre-cleaned by flushing the auxiliary water channel with water. Finally, the accessories are detached from the endoscope and placed in a fluid, such as water or a detergent solution. Typically, after the pre-cleaning procedure the endoscope is detached from the equipment (such as for example video system center, light source, suction pump) used in the patient procedure and transported to a reprocessing area.

In US 11445900B2 it was suggested to pre-clean an endoscope by using a plurality of input members in form of flexible tubes for use in delivering cleaning solution to multiple input ports of an endoscope, so that a cleaning solution is delivered through the flexible tubes to the endoscope.

However, known procedures for pre-cleaning are both relatively time-consuming and prone to errors. As the pre-cleaning typically consists of several subsequently conducted manually applied steps, the pre-cleaning is relatively time-consuming and if only one of these steps is not conducted exactly according to the pre-cleaning procedure, the endoscope might not be pre-cleaned sufficiently. Furthermore, because not all endoscope channels are connected to each other it is not possible to flush all channels with only one source. In typically conducted pre-cleaning procedures, the video system center is required to flush the air/water channel, a suction pump to aspire the suction and biopsy channel and a flushing pump or syringe to flush the auxiliary water channel. Therefore, the user is permanently involved in the procedure and needs to interact with several different user interfaces which requires that the user can operate all of these user interfaces. Additionally a lot of equipment/accessories is necessary to conduct the pre-cleaning procedure.

Furthermore, a problem is that after pre-cleaning, drying and solidification can occur in the channels that are no longer filled with fluid.

Furthermore, with known methods for pre-cleaning, it can be the case that not all channel sections, in particular branched channel sections, of the endoscope are flushed and thus channel sections, in particular branched channel sections, can be omitted by the pre-cleaning procedure. However, this can lead to insufficient pre-cleaning and to undesirable drying and solidification of residual organic debris.

### SUMMARY

Therefore, it is an object of the present invention to provide an improved method and an improved arrangement for pre-cleaning an endoscope. In particular, it is an object of the present invention to provide a method for pre-cleaning an endoscope that is less prone to errors.

According to a first aspect, it is provided a method for pre-cleaning an endoscope, preferably immediately or shortly after use of the endoscope, the method comprising: providing an endoscope that comprises a channel system, the channel system comprising: a first channel section, a second channel section and a third channel section, wherein these channel sections are connect to each other at a channel connection, and a first channel opening, a second channel opening and a third channel opening, wherein the first channel opening is arranged at an end of the first channel section and at a distal end of the endoscope, wherein the second channel opening is arranged at an end of the second channel section and formed as a connection port, in particular for connection of a fluid hose, and wherein the third channel opening is arranged at an end of the third channel section and formed as a connection port, in particular for connection of a fluid hose; connecting a liquid source, in particular a vessel that holds liquid, a suction device, preferably a suction pump, and a sealing element each to a different one of the first, second, and third channel opening such that each of the first, second, and third channel opening is connected to either the liquid source, the suction device, or the sealing element, wherein the liquid source is configured to provide a gas-tight sealing from the environment that surrounds the liquid source, wherein the liquid source has a holding chamber with a holding chamber volume for holding the liquid, wherein the holding chamber volume of the holding chamber is changeable; activating the suction device for drawing liquid from the liquid source towards the suction device, wherein the holding chamber volume of the liquid source reduces, preferably by the volume of liquid that is drawn out of the liquid source, during drawing of liquid from the liquid source.

In the following, initially the method for pre-cleaning an endoscope and its functions and advantageous embodiments will be described.

Preferably, the method is conducted immediately or shortly after use of the endoscope in a patient procedure. Preferably the method for pre-cleaning the endoscope is started within 60 minutes after being used in the patient procedure, particularly preferably within 30 minutes after being used in the patient procedure, in particular within 15 minutes after being used in the patient procedure. Preferably, the pre-cleaning is conducted at a patient bedside so that the method can be conducted immediately or shortly after use of the endoscope in a patient procedure without the need to move the endoscope to another location prior to conducting the pre-cleaning.

Preferably, the channel system comprises at least three channel sections. Preferably, the channel system can comprise more than three channel sections.

The first channel section, the second channel section, and the third channel section are connected to each other at the channel connection. Preferably, the channel connection is arranged inside of the endoscope.

Preferably, a channel section is to be understood as a part of a channel that extends from a channel opening to the channel connection or a channel that extends from a channel opening to the channel connection or a branched channel that extends from a channel opening to the channel connection. Preferably, the channel connection is understood to be a branching point where several channel sections, in particular the first, second and third channel section, merge.

The distal end of the endoscope may also be referred to as the distal tip of the endoscope. Preferably, the distal end is to be understood as the end portion of the endoscope that is located at the very front of an insert section of the endoscope, in particular the end portion that is first inserted into the patient when the insert section of the endoscope is moved into a patient. Preferably, outlets of channels of the endoscope are arranged at the distal end. Further preferably, a light guide is arranged at the distal end. Further preferably, an objective lens can be arranged at the distal end. It is particularly preferred if the distal end is arranged at the end of an insert section that has a flexible insertion tube.

Preferably, the connection ports are to be understood as ports to which fluid hoses or the like can be connected to.

Preferably, the endoscope can for example be a colonoscope, or a gastroscopes, or an ultrasonic endoscope, or a duodenoscopes, or another type of endoscope.

Preferably, the liquid source holds liquid that can be used to flush the channel sections. The liquid source has a holding chamber with a holding chamber volume for holding liquid. The holding chamber volume can change, in particular dependent on the volume of liquid that is arranged inside the holding chamber. Preferably, the holding chamber volume is substantially equal to the volume of liquid that is arranged in the holding chamber. Preferably, the holding chamber volume reduces corresponding to the reduction of liquid when liquid is drawn out of the liquid source.

Each of the first, second and third channel opening is connected to one of the liquid source, the suction device or the sealing element. Preferably, the liquid source is connected to the first channel opening, and the suction device is connected to the second channel opening, and the sealing element is connected to the third channel opening.

Preferably, by activating the suction device liquid is drawn from the liquid source through the first channel section and the second channel section towards the suction device.

Preferably, by activating the suction device air is drawn from the channel section to which the sealing element is connected to.

An advantage of this method is that air can be drawn out of the channel section to which the sealing element is connected to by applying an underpressure using the suction device, whereby an underpressure can build up in this channel section and the other channel sections are flushed with liquid, so that when in a further step the suction device is turned off and/or disconnected from the channel system due to the underpressure in the channel to which the sealing element is connected to liquid can be drawn into this channel section so that this channel section is also filled with the liquid. Thus, the first, second and third channel section can be filled with liquid by first applying an underpressure to the channel system and then removing and/or disconnecting the suction device to draw liquid into the channel section from which air has been drawn and in which an underpressure has built up. The channel section to which the sealing element is connected to is in particular filled with liquid because the underpressure reduces so that finally an ambient pressure is present.

A further advantage of this method is that the whole channel system, including the first, second and third channel section can be reliably filled with liquid. In particular, also branched channel sections can be reliably filled with liquid. In this way it can be ensured that all of the channel sections are sufficiently filled with liquid and pre-cleaned. Thus, insufficient pre-cleaning and undesirable drying and solidification of residual organic debris can be avoided.

An advantage of this method is that the channels can be kept humid and/or filled with liquid after having conducted the pre-cleaning procedure. Thus, drying and solidification in the channels after pre-cleaning can be prevented in a particularly advantageous way.

In a further preferred embodiment, the sealing element is connected to the corresponding channel opening in such a manner that this channel opening is sealed air-tight from the environment that surrounds the channel opening.

Preferably the sealing element is connected to the third channel opening. Such an air-tight sealing can be understood to be a gas-tight sealing.

An advantage of sealing the corresponding channel against the environment is that in this way no external gas can enter the channel and an underpressure can be built up in the corresponding channel, wherein this underpressure in the channel can later be used to draw liquid into the channel.

In a further preferred embodiment, the first channel section extends from the first channel opening to the channel connection, wherein the second channel section extends from the second channel opening to the channel connection, and wherein the third channel section extends from the third channel opening to the channel connection. Preferably, the first, second, and third channel section extend from the first, second, and third channel opening, respectively, to the channel connection.

In a further preferred embodiment, the first channel section and the second channel section form a channel that extends from the first channel opening to the second channel opening, and wherein the third channel section forms a branched channel section, which is fluidly connected to the channel at the channel connection. Such a channel formed by the first channel section and the second channel section can form a suction channel that extends from the first channel opening at the distal end of the endoscope to the second channel opening that forms a connection port to which the suction device can be connected.

In a further preferred embodiment, the liquid source is connected to the first channel opening and arranged at the distal end of the endoscope in such a manner that the liquid source is fluidly connected to the distal end of the endoscope and the liquid source and the distal end of the endoscope are sealed air-tight from the environment that surrounds the distal end of the endoscope and the liquid source.

An advantage of such an air-tight sealing of the distal end against the environment by using the liquid source is that in this way the holding chamber volume of the liquid source can decrease with liquid being drawn out of the liquid source.

Preferably, the liquid source and the distal end of the endoscope are also sealed liquid-tight from the environment that surrounds the distal end of the endoscope and the liquid source.

An advantage of sealing the distal end against the environment by using the liquid source is that in this way no external fluids can enter the channel sections and no liquid can flow out from the distal end into the environment that surrounds the liquid source.

In a further preferred embodiment, the suction device is connected to the second channel opening via an intermediate liquid holding element, in particular a hose, with a holding volume, which is preferably formed by a channel of the intermediate liquid holding element.

Preferably, such an intermediate liquid holding element is arranged between the second channel opening and the suction device. Preferably such an intermediate liquid holding element is configured to hold liquid that is drawn from the liquid source by applying an underpressure using the suction device towards the suction device. Preferably, after drawing liquid towards the suction device and into the intermediate liquid holding element, the suction device is turned off and/or disconnected so that no underpressure is applied by the suction device anymore and so that liquid arranged in the intermediate liquid holding element can be drawn to the third channel section by the underpressure that has built up in the third channel section.

An advantage of such an intermediate liquid holding element is that liquid can be collected and then provided to be transferred into the third channel section, wherein the liquid can automatically be transferred from the intermediate liquid holding element to the third channel section due to the underpressure that has built up in the third channel section. Thus, the third channel section can reliably be provided with the liquid so that the third channel section is pre-cleaned and no solidification processes can occur in the third channel section.

Preferably, the holding volume of the intermediate liquid holding element is equal to or larger than a holding volume of the third channel section. This way a sufficient amount of liquid can be collected in the intermediate liquid holding element to be transferred into the third channel section.

In a further preferred embodiment, by activating the suction device, air is removed from the third channel section in such a way that an underpressure builds up in the third channel section, wherein preferably the third channel section is formed as a biopsy branch or as an air channel branch.

Preferably, the extension of the third channel section from the third channel opening to the channel connection is several times smaller than the extension of the first channel section from the first channel opening to the channel connection. Preferably, the extension of the third channel section from the third channel opening to the channel connection is several times smaller than the extension of the second channel section from the second channel opening to the channel connection.

In a further preferred embodiment, the method comprises: venting the channel section to which the suction device is connected, and preferably other channel sections and/or channels, preferably by disconnecting the suction device from the second channel opening.

Preferably, venting the channel section to which the suction device is connected in particular stops the application of an underpressure by the suction device. Furthermore, venting the channel section to which the suction device is connected allows air to flow into the intermediate liquid holding element.

In a further preferred embodiment, the method comprises: after venting the channel section to which the suction device is connected, filling the third channel section with liquid from the channel section to which the suction device was connected by using an underpressure that was built up due to the underpressure applied in the third channel section by the suction device before venting.

Preferably, venting the channel section leads to a pressure difference between this channel and the third channel in which an underpressure is built up so that then liquid is drawn into the third channel section.

In a further preferred embodiment, connecting a sealing element to the third channel opening comprises arranging a transparent sealing element, preferably designed as a closed transparent tube element, onto the third channel opening.

Preferably, the sealing element is transparent. Such a transparent sealing element has the advantage that it can be seen from outside of the endoscope if liquid is arranged next to the sealing.

It is preferred that the sealing element is formed as a closed tube element that is preferably transparent or at least partly transparent. This way, liquid can be drawn through the third channel section into the sealing element and thus drawn completely through the third channel section. An advantage of such a tube-formed sealing element is that it can be verified easily from outside the endoscope by a user whether liquid is present in the sealing element and thus, whether liquid has been drawn through the third channel section.

In a further preferred embodiment, the liquid that is arranged in the liquid source and that is drawn through the channel sections is water or a disinfectant solution or a detergent solution.

It is preferred to use disinfectant solution and/or detergent solution in order to achieve a better removal of organic debris.

In a further preferred embodiment, the endoscope comprises the following channels: an air channel that extends from an air channel connection port to the distal end of the endoscope, a suction channel, preferably formed by the first and second channel section, that extends from a suction channel connection port, preferably formed by the second channel opening, to the distal end of the endoscope, a water channel that extends from a water channel connection port to the distal end of the endoscope, and preferably an auxiliary water channel that extends from an auxiliary water channel connection port to the distal end of the endoscope.

Preferably, the air channel is adapted to provide air to the distal end of the endoscope. Preferably, the suction channel is adapted to draw liquids and/or objects, such as for example tissue samples, from the distal end of the endoscope through the endoscope. Preferably, the water channel is adapted to provide water at the distal end of the endoscope. Preferably, the auxiliary water channel is adapted to provide water at the distal end of the endoscope.

Preferably, the channels are arranged parallel to each other over at least a part of their extension from one of the connection ports to the distal end of the endoscope. The endoscope can comprise at least one further channel sections, in particular branched channel sections such as a biopsy branch, that do not extend to the distal end of the endoscope.

In a further preferred embodiment, the channels are connected fluidly to each other at the distal end of the endoscope by arranging a container onto the distal end of the endoscope in such a manner that the distal end is sealed air-tight from the environment that surrounds the distal end. Preferably, before flushing channels of the endoscope, the container is not filled with liquid. An advantage of such an empty container is that liquid can be drawn from liquid sources that are arranged at connection ports through the channels to the container and from the container through at least a part of the channel system, in particular through the first channel section and the second channel section. Alternatively, it is also possible that the container is already filled with liquid before flushing the channels. Filling the container with liquid before starting the flushing of the channels can have the advantage that the distal end of the endoscope is in contact with liquid for a longer period of time and soaked for a longer period of time, which can be beneficial to achieve a more thorough pre-cleaning of the distal end, if needed.

Preferably, the inner volume of the container is not changeable. This can be achieved, for example, by using a relatively stiff material for the wall of the container that defines an inner chamber of the container. This way, when an underpressure is applied to the inner chamber of the container, the inner volume of the container does not contain but remains substantially unchanged so that the fluid connection of the channels at the end of the endoscope remains even when an underpressure is applied to the channel system and thus to the inner volume of the container.

In a further preferred embodiment, the method comprises: connecting liquid sources to at least two of the connection ports, preferably at least to the connection ports of the air channel and to the connection port of the water channel, and drawing liquid from the liquid sources via the channels of the endoscope, preferably via all of the channels of the endoscope, towards the suction device.

An advantage of such an embodiment is that liquid can be drawn through all of the channels to which such a liquid source is connected, and possibly through further channel sections and/or channels.

The advantages explained above with respect to the method for pre-cleaning are also advantages for such an arrangement for pre-cleaning.

According to a further aspect, it is provided an arrangement for pre-cleaning an endoscope, preferably immediately or shortly after use of the endoscope, in particular for pre-cleaning an endoscope according to the method as described herein, the arrangement comprising: an endoscope that comprises a channel system, the channel system comprising: a first channel section, a second channel section and a third channel section, wherein these channel sections are connected to each other at a channel connection, and a first channel opening, a second channel opening and a third channel opening, wherein the first channel opening is arranged at an end of the first channel section and at a distal end of the endoscope, wherein the second channel opening is arranged at an end of the second channel section and formed as a connection port, in particular for connection of a fluid hose, and wherein the third channel opening is arranged at an end of the third channel section and formed as a connection port, in particular for connection of a fluid hose, a liquid source, in particular a vessel that holds liquid, a suction device, preferably a suction pump, and a sealing element, wherein the liquid source, the suction device, and the sealing element are each connected to a different one of the first, second, and third channel opening such that each of the first, second and third channel opening is connected to either the liquid source, the suction device, or the sealing element, wherein the liquid source is configured to provide a gas-tight sealing from the environment that surrounds the liquid source, wherein the liquid source has a holding chamber with a holding chamber volume for holding the liquid, wherein the holding chamber volume of the holding chamber is changeable.

In a further preferred embodiment, the first channel section extends from the first channel opening to the channel connection, wherein the second channel section extends from the second channel opening to the channel connection, and wherein the third channel section extends from the third channel opening to the channel connection.

In a further preferred embodiment, the liquid source is connected to the first channel opening and arranged at the distal end of the endoscope in such a manner that the liquid source is fluidly connected to the distal end of the endoscope and the liquid source and the distal end of the endoscope are sealed air-tight from the environment that surrounds the distal end of the endoscope and the liquid source.

In a further preferred embodiment, the sealing element is connected to the corresponding channel opening in such a manner that this channel opening is sealed air-tight from the environment that surrounds the connection port, wherein preferably the sealing element is connected to the third channel opening.

In a further preferred embodiment, the sealing element is formed as a transparent sealing element, preferably designed as a closed transparent tube element that is connected to the third channel opening.

In a further preferred embodiment, the channels comprise an air channel that extends from an air channel connection port to the distal end of the endoscope, a suction channel, preferably formed by the first and second channel section, that extends from a suction channel connection port, preferably formed by the second channel opening, to the distal end of the endoscope, a water channel that extends from a water channel connection port to the distal end of the endoscope, and preferably an auxiliary water channel that extends from an auxiliary water channel connection port to the distal end of the endoscope.

As to the advantages, preferred embodiments and details of the individual different aspects and their preferred embodiments, reference is also made to the corresponding advantages, preferred embodiments and details described with reference to the respective other aspects.

Further advantageous embodiments result from the combination of individual, several or all of the preferred features described herein.

### BRIEF DESCRIPTION OF THE DRAWINGS

- Fig. 1:: shows a schematic depiction of an exemplary embodiment of an arrangement for pre-cleaning an endoscope;
- Fig. 2a:: shows a schematic flow diagram of a first part of an exemplary embodiment of a method for pre-cleaning an endoscope;
- Fig. 2b:: shows a schematic flow diagram of a second part of the embodiment of a method for pre-cleaning an endoscope shown in Fig. 2a;
- Fig. 3a:: shows a schematic depiction of an exemplary embodiment of an arrangement for pre-cleaning an endoscope;
- Fig. 3b:: shows a schematic depiction of an exemplary embodiment of an arrangement for pre-cleaning an endoscope;
- Fig. 4:: shows a schematic depiction of an exemplary embodiment of an arrangement for pre-cleaning an endoscope;
- Fig. 5:: shows a schematic flow diagram of an exemplary embodiment of a method for pre-cleaning an endoscope.

### DETAILED DESCRIPTION

In the figures, elements with the same or comparable functions are indicated with the same reference numerals.

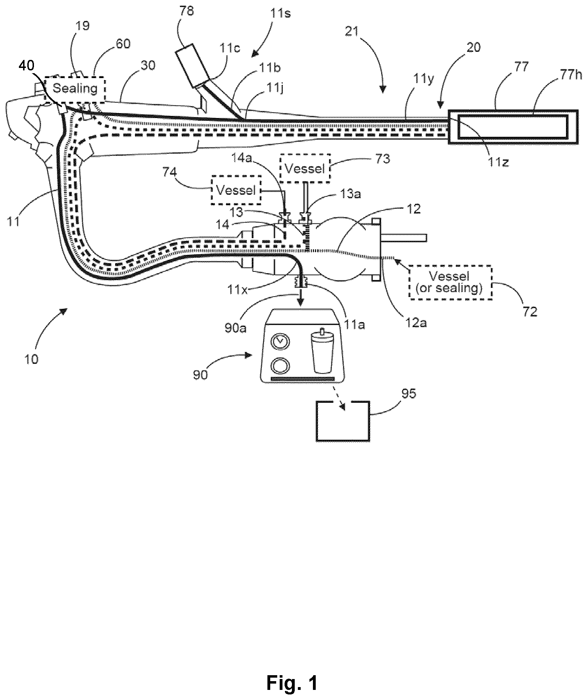

Fig. 1 shows a schematic depiction of an exemplary embodiment of an arrangement for pre-cleaning an endoscope 10. The endoscope 10 can be pre-cleaned immediately or shortly after use of the endoscope 10. The endoscope 10 comprises an endoscope body 30 and channels 11, 12, 13, 14 for transporting fluids, namely a suction channel 11, an air channel 12, a water channel 13, and an auxiliary water channel 14.

The suction channel 11 is adapted to draw liquids and/or objects, such as for example a tissue sample, from the distal end 20 of the endoscope 10 through the endoscope 10. The suction channel 11 extends from a second channel opening 11a that is formed as a suction channel connection port 11a to a first channel opening 11z that is arranged at the distal end 20 of the endoscope 10. The distal end 20 is the end portion of the endoscope 10 that is located at the very front of an insert section 21 of the endoscope 20.

The suction channel 11 is formed by a first channel section 11y and a second channel section 11x. The first channel section 11y extends from the first channel opening 11z to a channel connection 11j. The second channel section 11x extends from the second channel opening 11a to the channel connection 11j.

The endoscope 10 furthermore comprises a third channel section 11b that is formed as a biopsy branch 11b that extends from a third channel opening 11c that is formed as a biopsy branch connection port 11c to the channel connection 11j where it merges with the first channel section 11y and the second channel section 11x.

The first channel section 11y, the second channel section 11x and the third channel section 11b that merge at the channel connection 11j form the channel system 11s.

The air channel 12 is adapted to provide air to the distal end 20 of the endoscope 10. The air channel 12 extends from an air channel connection port 12a to the distal end 20 of the endoscope 10. The water channel 13 is adapted to provide water at the distal end 20 of the endoscope 10. The water channel 13 extends from a water channel connection port 13a to the distal end 20 of the endoscope 10. The auxiliary water channel 14 is adapted to provide water at the distal end 20 of the endoscope 10. The auxiliary water channel 14 extends from an auxiliary water channel connection port 14a to the distal end 20 of the endoscope 10.

The connection ports 11a, 11c, 12a, 13a, 14a are adapted for connection of fluid hoses and/or vessels and/or closure elements and/or sealing elements. Each of the channels 11, 12, 13, 14 extends from one of the connection ports 11a, 12a, 13a, 14a to the distal end 20 of the endoscope 10.

The air channel 12 and the water channel 13 can be fluidly connected and/or separated from each other via an air/water cylinder 19. A vent can be assembled to the air/water cylinder 19. The air/water cylinder 19 is sealed with a closure element 60 that is adapted to close the air/water cylinder 19 fluid-tight. Furthermore, a suction cylinder 40 is provided to which the suction channel 11 is connected to. A vent can be assembled to the suction cylinder 40.

A sealing element 78 is arranged onto the biopsy branch connection port 11c. The sealing element 78 is formed as a transparent sealing element that is designed as a closed transparent tube element. The sealing element 78 is arranged onto the third channel opening 11c in such a manner that the third channel opening 11c is sealed air-tight from the environment that surrounds the third channel opening 11c.

A liquid source 77 that is formed as a vessel is arranged onto the distal tip 20 of the endoscope 10 so that the liquid source 77 is connected to the first channel opening 11z and thus to the first channel section 11y. The liquid source 77 has a liquid holding chamber 77 h with a holding chamber volume that is changeable. Liquid to be drawn into the channel system 11s is arranged in the liquid holding chamber 77h, wherein the volume of the liquid equals the liquid holding volume. The liquid source 77 is sealed air-tight and liquid-tight towards the environment that surrounds the liquid source 77.

A suction device 90 that is formed as a suction pump in this preferred embodiment is connected via a fluid hose 90a to the connection port 11a of the second channel section 11x. A suction jar 95 can be connected to the suction device 90 to collect liquid drawn out of the channel system 11s. When the suction device 90 is activated, an underpressure is applied to the channel system 11s so that liquid is drawn from the liquid source 77 through the first channel section 11y and the second channel section 11x towards the suction device 90. During drawing liquid from the liquid source 77, the liquid volume and at the same time the volume of the liquid holding chamber 77h reduces, because the liquid source is formed air-tight so that no air can enter the liquid holding chamber from outside of the liquid source. Due to the underpessure in the channel system 11s applied by the suction device air is drawn out of the third channel section 11b and possibly from the sealing element 78 towards the suction device 90 so that an underpressure builds up in the third channel section 11b.

When the liquid is completely withdrawn from the liquid source, i.e. when the liquid holding chamber is emptied and the volume of the liquid chamber 77h is substantially equal to zero, the suction device 90 is disconnected from the fluid hose 90a so that no underpressure is applied by the suction device 90 anymore and that the channel system 11s is vented as the channel system 11s is opened against the environment at the second channel opening 11a and/or at the fluid hose 90a. Now the lowest pressure is present in the third channel section 11b to which an underpressure was applied by removing air from the third channel section 11b before. Due to the lowest pressure the liquid that is present in the channel system 11s and/or in the fluid hose 90a is drawn towards the third channel section 11b and into the third channel section 11b.

If the fluid hose 90a is formed as an intermediate liquid holding element, the holding volume of this intermediate liquid holding element is equal to or larger than a holding volume of the third channel section 11b. This way a sufficient amount of liquid can be collected in the intermediate liquid holding element 90a to be transferred into the third channel section 11b and preferably the whole channel system 11s can be kept humid and/or filled with liquid.

Furthermore, additional liquid sources, preferably formed as vessels, or closure elements to close connection ports can be arranged onto additional connection ports as follows: A vessel 72 can be arranged onto the connection port 12a of the air channel 12. If the air channel 12 does not need to be flushed during the pre-cleaning procedure, a closure element can be arranged onto the connection port 12a instead of the vessel 72. A vessel 73 can be arranged onto the connection port 13a of the water channel 13. If the water channel 13 does not need to be flushed during the pre-cleaning procedure, a closure element can be arranged onto the connection port 13a instead of the vessel 73. Furthermore, a vessel 74 can be arranged onto the connection port 14a of the auxiliary water channel 14. If auxiliary water channel 14 does not need to be flushed during the pre-cleaning procedure, a closure element can be arranged onto the connection port 14a instead of the vessel 74. The vessels 72, 73, 74 can be filled with liquid, in particular with a disinfectant solution and/or detergent solution.

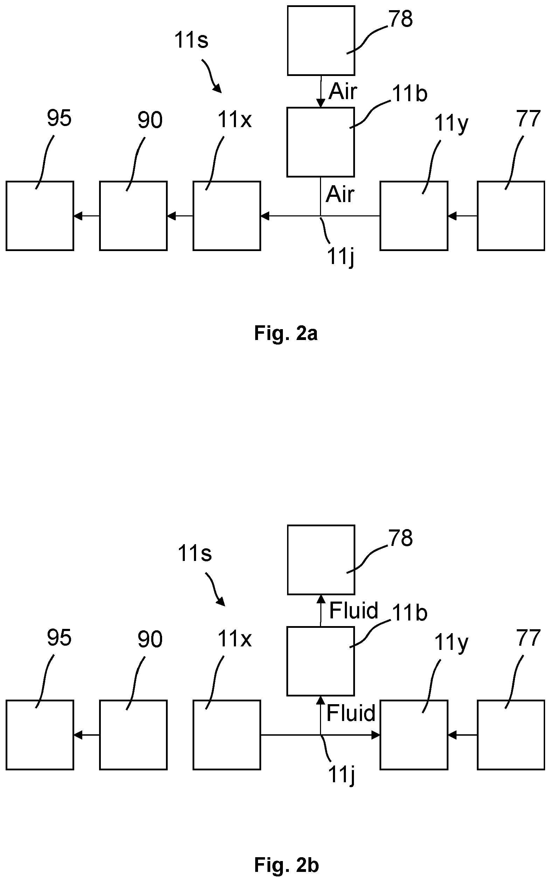

Fig. 2a shows a schematic flow diagram of a first part of an exemplary embodiment of a method for pre-cleaning an endoscope. Channel system 11s, sealing element 78, liquid source 77, suction pump 90 and suction jar 95 can be arranged as described in connection with Fig. 1. The channel system 11s comprises a first channel section 11y, a second channel section 11x and a third channel section 11b, wherein the first, second and third channel section are connected at a channel connection 11j. A suction pump 90 is connected to the channel system 11s at a second channel opening of the second channel section 11x so that an underpressure is applied to the channel system 11s. Due to this underpressure, liquid is drawn out of the vessel 77 through the first channel section 11y and through the second channel section 11x towards the suction pump 90. The liquid or a part of the liquid can be collected in a suction jar 95. Due to the underpressure applied to the channel system 11s, air is drawn out of the third channel section 11b and the sealing element 78. Both the liquid and air flow directions are indicated by arrows.

Fig. 2b shows a schematic flow diagram of a second part of the embodiment of a method for pre-cleaning an endoscope shown in Fig. 2a.

After having drawn the liquid out of the vessel 77 and through the first and second channel sections 11y, 11x as described in connection with Fig. 2a, the suction pump 90 is disconnected from the channel system 11s by disconnecting the suction pump 90 from the second channel opening of the second channel section 11x so that no underpressure is applied to the channel system 11s by the suction device 90 anymore. Due to the underpressure that has built up in the third channel section 11b, liquid is drawn from the second channel section 11x towards the third channel section 11b so that the third channel section 11b is filled with liquid. The liquid flow directions are indicated by arrows.

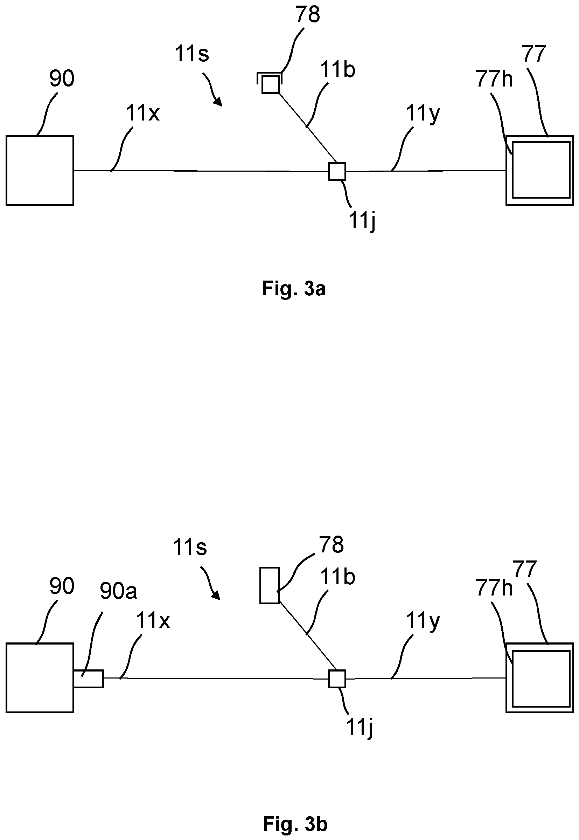

Fig. 3a shows a schematic depiction of an exemplary embodiment of an arrangement for pre-cleaning an endoscope that can be used in a method as described in connection with Fig. 2a and Fig. 2b. The channel system 11s comprises a first channel section 11y that is a part of the suction channel, a second channel section 11x that is also a part of the suction channel, and a third channel section 11b that is formed as a biopsy branch, wherein the first, second and third channel section are connected at a channel connection 11j. A suction pump 90 is connected to the channel system 11s at a second channel opening of the second channel section 11x so that an underpressure can be applied to the channel system 11s. The vessel 77 that is connected to the channel opening of the first channel section 11y is formed air-tight and has a liquid holding chamber 77h that has a changeable volume. A sealing element 78 is arranged onto the third channel opening of the third channel section 11b in such a manner that the third channel opening is sealed air-tight from the environment that surrounds the third channel opening. Liquid can be drawn through the channel sections as described in connection with Fig. 2a and Fig. 2b.

Fig. 3b shows a schematic depiction of an exemplary embodiment of an arrangement for pre-cleaning an endoscope that is similar to the embodiment shown in Fig. 3a and thus it is referred to the description of Fig. 3a. However, in the embodiment shown in Fig. 3b, the suction device 90 is connected to the second channel opening of the second channel 11x via an intermediate liquid holding element 90a that is formed as a fluid hose 90a that has a holding volume, which is formed by a channel of the intermediate liquid holding element. After disconnecting the suction pump 90 from the channel system 11s, liquid can be drawn from the intermediate liquid holding element 90a through the second channel section 11x and into the third channel section 11b. Furthermore, the sealing element 78 that is arranged onto the biopsy branch 11b is formed as a transparent sealing element that is designed as a closed transparent tube element 78 that is arranged onto the third channel opening of the third channel section 11b in such a manner that the third channel opening is sealed air-tight from the environment that surrounds the third channel opening.

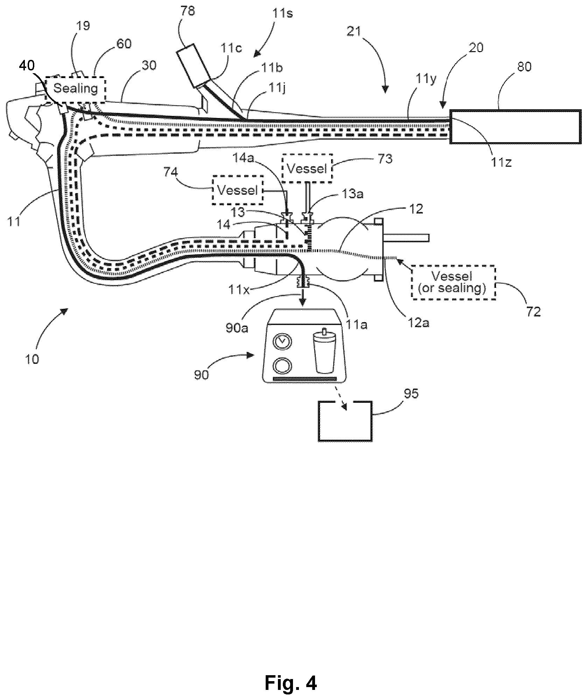

Fig. 4 shows a schematic depiction of an exemplary embodiment of an arrangement for pre-cleaning an endoscope 10. The arrangement is substantially configured and arranged as described in connection with Fig. 1. However, instead of a liquid holding element, a container 80 is arranged onto the distal tip 20 of the endoscope 10.

The channels 11, 12, 13, 14 are fluidly connected to each other at the distal end 20 of the endoscope 10 by arranging the container 80 onto the distal end 20 of the endoscope 10. The container 80 is arranged onto the distal end 20 of the endoscope 10 in such a manner that the distal end 20, including the outer circumferential surface 20a of the distal end 20, is arranged entirely inside the container 80. If the container 80 gets filled with liquid, the whole distal end 20, including the outer surface of the distal end 20, is in contact with the liquid so that a reliable pre-cleaning of the whole distal end 20 can be achieved. The container 80 is arranged on the distal end 20 of the endoscope 10 in such a manner that the distal end 20 of the endoscope 10 is sealed liquid-tight and gas-tight from the environment that surrounds the container 80.

A vessel 72 is arranged onto the connection port 12a of the air channel 12. If the air channel 12 does not need to be flushed during the pre-cleaning procedure, a closure element can be arranged onto the connection port 12a instead of the vessel 72. A vessel 73 is arranged onto the connection port 13a of the water channel 13. If the water channel 13 does not need to be flushed during the pre-cleaning procedure, a closure element can be arranged onto the connection port 13a instead of the vessel 73. Furthermore, a vessel 74 is arranged onto the connection port 14a of the auxiliary water channel 14. If auxiliary water channel 14 does not need to be flushed during the pre-cleaning procedure, a closure element can be arranged onto the connection port 14a instead of the vessel 74. The vessels 72, 73, 74 are filled with liquid, in particular with a disinfectant solution and/or detergent solution.

When the suction device 90 is activated, an underpressure is applied to the system so that liquid is drawn towards the suction device 90 as described in detail below.

Liquid is drawn from the vessels 72, 73, 74 through the corresponding channels 12, 13, 14 towards the distal end 20. Due to the fluid connection at the distal end 20 that is provided by the container 80 arranged at the distal end 20, the liquid is drawn from there through the whole extension of the suction channel 11, out of the connection port 11a, via the fluid hose 90a to the suction device 90 and from the suction device 90 to the suction jar 95.

When the liquid is completely withdrawn from the vessels 72, 73, 74, the suction device 90 is disconnected from the fluid hose 90a so that no underpressure is applied by the suction device 90 anymore and that the channel system 11s is vented as the channel system 11s is opened against the environment at the second channel opening 11a and/or at the fluid hose 90a. Now the lowest pressure is present in the third channel section 11b to which an underpressure was applied by removing air from the third channel section 11b before. Due to the lowest pressure the liquid that is present in the channel system 11s and/or in the fluid hose 90a is drawn towards the third channel section 11b and into the third channel section 11b.

If the fluid hose 90a is formed as an intermediate liquid holding element, the holding volume of this intermediate liquid holding element is equal to or larger than a holding volume of the third channel section 11b. This way a sufficient amount of liquid can be collected in the intermediate liquid holding element 90a to be transferred into the third channel section 11b and preferably the whole channel system 11s can be kept humid and/or filled with liquid.

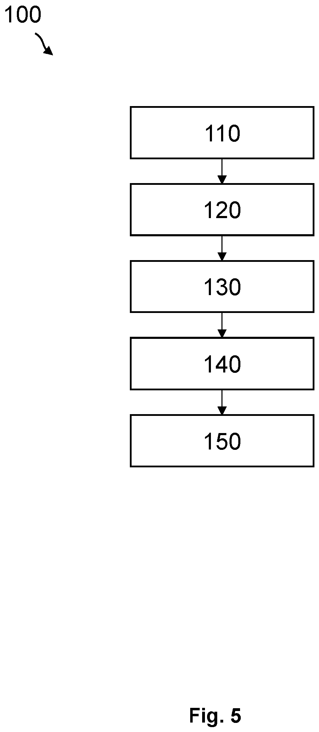

Fig. 5 shows a schematic flow diagram of an exemplary embodiment of a method 100 for pre-cleaning an endoscope. The method 100 is conducted immediately or shortly after use of the endoscope in a patient procedure and comprises the following steps:
In a step 110, providing an endoscope that comprises a channel system, the channel system comprising: a first channel section, a second channel section and a third channel section, wherein these channel sections are connect to each other at a channel connection, and a first channel opening, a second channel opening and a third channel opening, wherein the first channel opening is arranged at an end of the first channel section and at a distal end of the endoscope, wherein the second channel opening is arranged at an end of the second channel section and formed as a connection port, in particular for connection of a fluid hose, and wherein the third channel opening is arranged at an end of the third channel section and formed as a connection port, in particular for connection of a fluid hose.

In a step 120, connecting a liquid source, in particular a vessel that holds liquid, a suction device, preferably a suction pump, and a sealing element each to a different one of the first, second, and third channel opening such that each of the first, second, and third channel opening is connected to either the liquid source, the suction device, or the sealing element, wherein the liquid source is configured to provide a gas-tight sealing from the environment that surrounds the liquid source, wherein the liquid source has a holding chamber with a holding chamber volume for holding the liquid, wherein the holding chamber volume of the holding chamber is changeable.

In a step 130, activating the suction device for drawing liquid from the liquid source towards the suction device, wherein the holding chamber volume of the liquid source reduces, preferably by the volume of liquid that is drawn out of the liquid source, during drawing of liquid from the liquid source.

In a step 140, venting the channel section to which the suction device is connected, and preferably other channel sections and/or channels, preferably by disconnecting the suction device from the second channel opening.

In a step 150, after venting the channel section to which the suction device is connected, filling the third channel section with liquid from the channel section to which the suction device was connected by using an underpressure that was built up due to the underpressure applied in the third channel section by the suction device before venting.

## Claims

1. A method for pre-cleaning an endoscope, preferably immediately or shortly after use of the endoscope, the method comprising:
- providing an endoscope that comprises a channel system, the channel system comprising:
∘ a first channel section, a second channel section and a third channel section, wherein these channel sections are connect to each other at a channel connection, and
∘ a first channel opening, a second channel opening and a third channel opening,
▪ wherein the first channel opening is arranged at an end of the first channel section and at a distal end of the endoscope,
▪ wherein the second channel opening is arranged at an end of the second channel section and formed as a connection port, in particular for connection of a fluid hose, and
▪ wherein the third channel opening is arranged at an end of the third channel section and formed as a connection port, in particular for connection of a fluid hose;
- connecting a liquid source, in particular a vessel that holds liquid, a suction device, preferably a suction pump, and a sealing element each to a different one of the first, second, and third channel opening such that each of the first, second, and third channel opening is connected to either the liquid source, the suction device, or the sealing element, wherein the liquid source is configured to provide a gas-tight sealing from the environment that surrounds the liquid source, wherein the liquid source has a holding chamber with a holding chamber volume for holding the liquid, wherein the holding chamber volume of the holding chamber is changeable;
- activating the suction device for drawing liquid from the liquid source towards the suction device, wherein the holding chamber volume of the liquid source reduces, preferably by the volume of liquid that is drawn out of the liquid source, during drawing of liquid from the liquid source.

2. The method according to the preceding claim,
wherein the sealing element is connected to the corresponding channel opening in such a manner that this channel opening is sealed air-tight from the environment that surrounds the channel opening,
wherein preferably the sealing element is connected to the third channel opening.

3. The method according to at least one of the preceding claims,
wherein the first channel section extends from the first channel opening to the channel connection, wherein the second channel section extends from the second channel opening to the channel connection, and wherein the third channel section extends from the third channel opening to the channel connection, and/or
wherein the first channel section and the second channel section form a channel that extends from the first channel opening to the second channel opening, and wherein the third channel section forms a branched channel section, which is fluidly connected to the channel at the channel connection.

4. The method according to at least one of the preceding claims,
wherein the liquid source is connected to the first channel opening and arranged at the distal end of the endoscope in such a manner that the liquid source is fluidly connected to the distal end of the endoscope and the liquid source and the distal end of the endoscope are sealed air-tight from the environment that surrounds the distal end of the endoscope and the liquid source.

5. The method according to at least one of the preceding claims,
wherein the suction device is connected to the second channel opening via an intermediate liquid holding element, in particular a hose, with a holding volume, which is preferably formed by a channel of the intermediate liquid holding element,
wherein preferably the holding volume of the intermediate liquid holding element is larger than a holding volume of the branched channel section.

6. The method according to at least one of the preceding claims,
wherein by activating the suction device, air is removed from the third channel section in such a way that an underpressure builds up in the third channel section, wherein preferably the third channel section is formed as a biopsy branch or as an air channel branch.

7. The method according to at least one of the preceding claims, comprising:
- venting the channel section to which the suction device is connected, and preferably other channel sections and/or channels, preferably by disconnecting the suction device from the second channel opening, and preferably the method comprising:
- after venting the channel section to which the suction device is connected, filling the third channel section with liquid from the channel section to which the suction device was connected by using an underpressure that was built up due to the underpressure applied in the third channel section by the suction device before venting.

8. The method according to at least one of the preceding claims,
wherein connecting a sealing element to the third channel opening comprises arranging a transparent sealing element, preferably designed as a closed transparent tube element, onto the third channel opening.

9. The method according to at least one of the preceding claims,
wherein the liquid that is arranged in the liquid source and that is drawn through the channel sections is water or a disinfectant solution or a detergent solution.

10. The method according to at least one of the preceding claims,
wherein the endoscope comprises the following channels:
- an air channel that extends from an air channel connection port to the distal end of the endoscope,
- a suction channel, preferably formed by the first and second channel section, that extends from a suction channel connection port, preferably formed by the second channel opening, to the distal end of the endoscope,
- a water channel that extends from a water channel connection port to the distal end of the endoscope,
- and preferably an auxiliary water channel that extends from an auxiliary water channel connection port to the distal end of the endoscope.

11. An arrangement for pre-cleaning an endoscope, preferably immediately or shortly after use of the endoscope, in particular for pre-cleaning an endoscope according to the method according to at least one of the preceding claims, the arrangement comprising:
- an endoscope that comprises a channel system, the channel system comprising:
∘ a first channel section, a second channel section and a third channel section, wherein these channel sections are connected to each other at a channel connection, and
∘ a first channel opening, a second channel opening and a third channel opening,
▪ wherein the first channel opening is arranged at an end of the first channel section and at a distal end of the endoscope,
▪ wherein the second channel opening is arranged at an end of the second channel section and formed as a connection port, in particular for connection of a fluid hose, and
▪ wherein the third channel opening is arranged at an end of the third channel section and formed as a connection port, in particular for connection of a fluid hose,
- a liquid source, in particular a vessel that holds liquid,
- a suction device, preferably a suction pump, and
- a sealing element,
wherein the liquid source, the suction device, and the sealing element are each connected to a different one of the first, second, and third channel opening such that each of the first, second and third channel opening is connected to either the liquid source, the suction device, or the sealing element,
wherein the liquid source is configured to provide a gas-tight sealing from the environment that surrounds the liquid source, wherein the liquid source has a holding chamber with a holding chamber volume for holding the liquid, wherein the holding chamber volume of the holding chamber is changeable.

12. The arrangement according to the preceding claim,
wherein the first channel section extends from the first channel opening to the channel connection, wherein the second channel section extends from the second channel opening to the channel connection, and wherein the third channel section extends from the third channel opening to the channel connection.

13. The arrangement according to at least one of the preceding claims,
wherein the liquid source is connected to the first channel opening and arranged at the distal end of the endoscope in such a manner that the liquid source is fluidly connected to the distal end of the endoscope and the liquid source and the distal end of the endoscope are sealed air-tight from the environment that surrounds the distal end of the endoscope and the liquid source.

14. The arrangement according to at least one of the preceding claims,
wherein the sealing element is connected to the corresponding channel opening in such a manner that this channel opening is sealed air-tight from the environment that surrounds the connection port, wherein preferably the sealing element is connected to the third channel opening, and/or wherein the sealing element is formed as a transparent sealing element, preferably designed as a closed transparent tube element, that is connected to the third channel opening.

15. The arrangement according to the at least one of the preceding claims,
wherein the channels comprise
- an air channel that extends from an air channel connection port to the distal end of the endoscope,
- a suction channel, preferably formed by the first and second channel section, that extends from a suction channel connection port, preferably formed by the second channel opening, to the distal end of the endoscope,
- a water channel that extends from a water channel connection port to the distal end of the endoscope,
- and preferably an auxiliary water channel that extends from an auxiliary water channel connection port to the distal end of the endoscope.
